# EUROPEAN PATENT SPECIFICATION

(11) **EP 1 641 944 B1**
(45) Date of publication and mention of the grant of the patent: **29.06.2011**
(21) Application number: 04756925.6
(22) Date of filing: 09.07.2004
(51) Int. Cl.: C12Q 1/68

(54) **ROOM TEMPERATURE ELUTION OF NUCLEIC ACIDS**
RAUMTEMPERATUR-ELUIERUNG DER NUKLEINSÄUREN
ELUTION D'ACIDES NUCLEIQUES A TEMPERATURE AMBIANTE

(30) Priority: 09.07.2003 US 485972 P
(43) Date of publication of application: 05.04.2006
(73) Proprietor: GENVAULT CORPORATION, Carlsbad, CA 92011 (US)
(72) Inventor: DAVIS, James, C., Carlsbad, CA 92009 (US); HOGAN, Michael, Tucson, AZ 85716 (US)
(74) Representative: Vossius & Partner
(86) International application number: PCT/US2004/022391
(87) International publication number: WO 2005/007852

(56) References cited:
- EP-A- 0 389 063
- EP-A- 0 391 608
- EP-A- 0 832 897
- WO-A-92/18514
- KOHN K.W. ET AL: 'Fractionation of DNA from mammalian cells by alkaline elution.' BIOCHEMISTRY 19 OCT 1976 LNKD- PUBMED:9979 vol. 15, no. 21, 19 October 1976, pages 4629 - 4637 ISSN: 0006-2960
- MANDREKAR P. ET AL: 'Extraction and Isolation of DNA from Blood Cards and Buccal Swabs in a 96-well Format' PROMEGA APPLICATION NOTES, 01 January 2003, pages 1 - 2, XP007912840
- OZTEKIN N. ET AL: 'Adsorption of polyethyleneimine from aqueous solutions on bentonite clays' MATERIALS LETTERS, NORTH HOLLAND PUBLISHING COMPANY. AMSTERDAM, NL LNKD- DOI:10.1016/S0167-577X(01)00622-X vol. 55, no. 1-2, 01 July 2002, pages 73 - 76, XP004354306 ISSN: 0167-577X

## Description

### Related Applications

This application claims benefit of priority to U.S. Provisional Patent Application Serial No. 60/485,972 filed July 9, 2003.

### Field of the Invention

The present invention relates to recovery of nucleic acids from dried samples. In particular, the invention relates to room temperature recovery of DNA by applying solution at alkaline pH between 11 and 12 to a substrate having DNA-containing samples dried thereon, to remove DNA from the substrate and recover the DNA in solution phase.

### Background of the Invention

The DNA from biological samples such as blood, buccal swabs, tissue homogenates and other DNA source materials, can be stored or "archives" by applying a DNA-containing sample to a storage medium and allowing the sample to dry on the storage medium. Dry solid media for DNA storage are well known and have been commercially available for years. Examples of storage media include paper, *e.g.*, S&S 903 paper or IsoCode® paper (Schleicher and Schuell, Keene NH), or FTA® paper (Whatman, Inc., Newton MA). U.S. Patent No. (henceforth, "US") 6,322,983 and US 6,447,804 to Burgoyne disclose DNA storage on a solid matrix having a compound or composition which protects against degradation of DNA incorporated into or absorbed on the matrix. DNA can be eluted from samples on dry solid media and used for subsequent analysis.

Biological samples that have been stored for purposes other than DNA collection, *e.g.*, tissue samples that have been fixed in paraffin for histological analysis, contain DNA that can be extracted and used for subsequent analysis There are also naturally-occurring examples of storage media, *e.g.*, amber or fossilized (ossified) materials that can contain biological samples from which DNA can be extracted and used for subsequent analysis.

### Summary of the Invention

The present invention is defined by the claims.

The invention disclosed herein provides a method for eluting nucleic acid, in particular DN4, from a dried sample using an elution buffer with a pH of between 11 and 12. In particular, the invention provides a method for eluting DNA from a dried sample on storage media, wherein said storage media is a polyester material or a cellulosic material selected from the group consisting of S&S 903 paper, isoCode paper, FTA paper and Generation Capture Cards, and wherein the method is carried out at room temperature, avoids the use of toxic materials, and is automation-compatible while providing high-quality double stranded DNA suitable for numerous downstream applications.

The present invention provides a method for eluting nucleic acids, including DNA and RNA, from a nucleic acid-containing sample which has been dried on storage media, wherein said storage media is a polyester material or a cellulosic material selected from the group consisting of S&S 903 paper, IsoCode paper, FTA paper and Generation Capture Cards, and wherein the method includes the steps of providing a nucleic acid-containing sample on storage media incubating the sample on storage media at room temperature with an elution buffer having a pH of between 11.0 and 12.0, and eluting nucleic acid from the sample on storage media. The present invention provides an elution buffer having a pH of between about 11.4 and about 11.8.

The elution buffer may contain an organic buffering agent and/or an inorganic buffering agent. The elution buffer may contain Tris (hydroxymethyl) aminomethane hydrochloride (Tris) at a concentration of about 10 mM, having a pH of about 11.4, 11.5, 11.6, 11.7, or 11.8. The elution buffer, with a pH of between 11.0 and 12.0, may contain 4-(cyclohexylamino) -1-butanesulfonic acid (CABS), 3-(cyclohexylamino)-1-propanesulfonic acid (CAPS), 3-(cyclohexylamino-2-hydroxy-1-propanesulfonic acid (CAPSO), 2-(cyclohexylamino) ethanesulfonic acid (CHES), N-(2-hydroxyethyl)piperazine-N'-(3-propanesulfonic acid) (EPPS), N-(2-hydroxyethyl)piperazine-N'-(2-ethanesulfonic acid (HEPES), 2-(N-morpholino) ethanesulfonic acid (MES), 3-(N-morpholino) propanesulfonic acid (MOPS), piperazine-N,N'-bis (2-ethanesulfonic acid (PIPES), [(2-hydroxy-1,1-bis (bydroxymethyl]ethyl) amino]-1-propanesulfonic acid (TAPS), ethanolamine, 3-amino-1-propanesulfonic acid.

The present invention further provides an optional wash step, including the step of contacting the sample on storage media prior with wash buffer prior to contacting the sample with elution buffer. The wash buffer can contain a buffering agent and a chelating agent. The wash buffer can further contain a detergent. The wash buffer can be 10 mM Tris and 0.1 mM EDTA at a pH of about 8.0, and may optionally contain a detergent such as 1 % Triton X-100 or Tween 20. The wash buffer can contain one or more enzyme such as proteases, in particular proteinase K, and may contain lysis buffer or digestion buffer.

Also described herein are kits for nucleic acid from a nucleic acid-containing sample on storage media, where the kits contain an elution buffer having a pH of between about 11.0 and about 12.0 and instructions eluting nucleic acid from a. sample on storage media. The elution buffer has a pH of between about 11.4 and about 11.8, and may contain an organic buffering agent and/or an inorganic buffering agent. The elution buffer may contain Tris (hydroxymethyl) aminomethane hydrochloride (Tris) at a concentration of about 10 mM, having a pH of about 11.4, 11.5, 11.6, 11.7, or 11.8. The elution buffer, with a pH of between about 11.0 and about 12.0, may contain 4-(cyclohexylamino) -1-butanesulfonic acid (CABS), 3-(cyclohexylamino)-1-propanesulfonic acid (CAPS), 3-(cyclohexylamino-2-hydroxy-1-propanesulfonic acid (CAPSO), 2-(cyclohexylamino) ethanesulfonic acid (CHES), N-(2-hydroxyethyl)piperazine-N'-(3-propanesulfonic acid) (EPPS), N-(2-hydroxyethyl)piperazine-N'-(2-ethanesulfonic acid (HEPES), 2-(N-morpholino) ethanesulfonic acid (MES), 3-(N-morpholino) propanesulfonic acid (MOPS), piperazine-N,N'-bis (2-ethanesulfonic acid (PIPES), [(2-hydroxy-1,1-bis [bydroxymethyl]ethyl) amino]-1-propanesulfonic acid (TAPS), ethanolamine, 3-amino-1-propanesulfonic acid.

The kits also described herein may further contain a wash buffer. The wash buffer can contain a buffering agent and a chelating agent. The wash buffer can further contain a detergent. The wash buffer can be 10 mM Tris and 0.1 mM EDTA at a pH of about 8.0, and may optionally contain a detergent such as 1% Triton X-100 or Tween 20. The wash buffer can contain one or more enzyme such as protease in particular proteinase K, and may contain lysis buffer or digestion buffer. The kits also described herein may further contain means for recovering the eluted nucleic acid, including DNA and RNA. The kits also described herein may contain means for analyzing the eluted nucleic acid.

### Brief Description of the Drawings

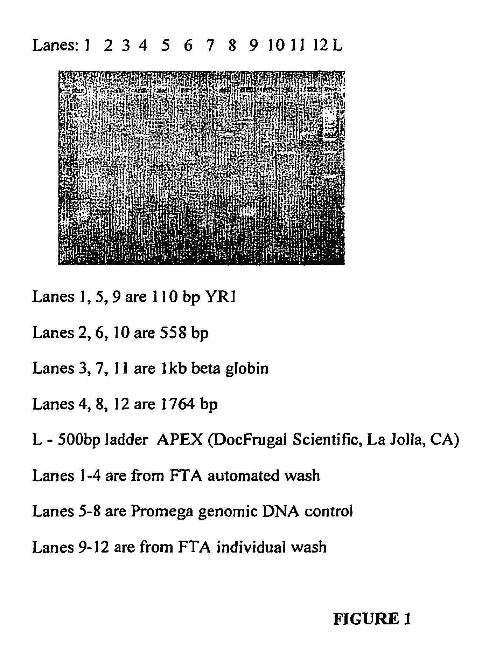

**Figure 1** shows an image of an agarose gel of PCR products obtained using DNA eluted from 10 µl of human blood from FTA® storage media, where two modifications of the GenVault room temperature elution method were used and single set of PCR primers was used in each lane: Lanes 1-4 show PCR products from samples eluted using an automated wash protocol, and Lanes 9-12 show PCR products from samples eluted using the standard "one element per column" wash protocol; Lanes 5-8 are genomic DNA control samples; Lane "L" on the far right is a 500 bp ladder.
**Figure 2** shows DNA yields obtained using an elution buffer containing 10 mM Tris adjusted to different pH values; plotted results are analyzed using one-way analysis, with a paired Student's t test of each pair of results (far right).
**Figure 3** shows the DNA yields obtained using elution buffers containing a 10 mM solution of different buffers adjusted to different pH values; results are shown as ng of DNA obtained from two rounds of elution.
**Figure 4** shows two examples of GenVault GenCode sample identifiers eluted from elements using methods disclosed herein; each GenCode was applied to the element prior to addition of 10 µl of human blood, and elements were washed with the standard protocol and eluted with 10 mM Tris at pH 11.6.

**Table 1** shows the DNA yield from 10 µl of human blood on FTA® storage media, using 10mM Tris elution buffer adjusted to various pH values from 11.4 to 11.8.

**Table 2** shows the DNA yields from human blood on FTA® elements using CST™ and MagneSil™ magnetic beads to purify DNA eluted from the elements using proteinase K digestion and the RT elution protocol.

**Table 3** shows the DNA yields from human blood on FTA® elements using Qiagen columns to purify DNA eluted from FTA® elements using the RT elution protocol.

**Table 4** shows the DNA yields from human blood on FTA® elements using phenol:chloroform extraction to purify DNA eluted from FTA® elements using the RT elution protocol.

**Table 5** shows the DNA yields from human blood in GTA® elements using Gentra Generation™ cartridges to purify DNA eluted from FTA® elements using the RT elution protocol; Elements 1 and 2 were treated with proteinase K prior to elution and purification, while Elements 3 and 4 were not treated with proteinase K prior to elution and purification.

### Detailed Description of the Invention

The present invention provides methods for efficient recovery of nucleic acids, in particular DNA, from storage media, wherein said storage media is a polyester material or a cellulosic material selected from the group consisting of S&S 903 paper, IsoCode paper, FTA paper and Generation Capture Cards. The present invention provides a method for using alkaline elution buffer having a pH between 11 and 12 to elute nucleic acids from dried samples on said storage media at room temperature. Briefly, the storage media is incubated with an elution buffer having an alkaline pH between 11 and 12, after which the nucleic acid separates from the storage media and is released into solution. Optionally, the storage media having a nucleic acid-containing sample is first washed with a suitable buffer one or more times prior to incubation with elution buffer. During incubation with the elution agent, the storage media is optionally subjected to moderate mechanical agitation to enhance release of nucleic acid into solution. Preferably, the eluted nucleic acid is recovered. The eluted nucleic acid is suitable for numerous downstream applications.

In accordance with one aspect, the present invention provides a method for room temperature elution of DNA, including double stranded DNA (dsDNA) and/or single stranded (ssDNA), from the storage media. Preferably, the eluted DNA is recovered. The eluted DNA is suitable for numerous downstream applications including but not limited to, polymerase chain reaction (PCR) based applications, short tandem repeat (STR) marker analysis, single nucleotide polymorphism (SNP) analysis, and whole genome amplification (WGA).

In accordance with another aspect, the present invention provides a method for room temperature elution of RNA from the storage media. Preferably, the eluted RNA is recovered. The eluted RNA is suitable for numerous downstream applications including but not limited to reverse transcription, reverse-transcription-polymerase chain reaction (RT-PCR), amplification, sequencing, and hybridization reactions including Northern blotting.

"Storage media" in accordance with the invention refers to cellulosic material (paper), selected from the group consisting of S&S 903 paper or IsoCode® paper (Schleicher and Schuell, Keene NH), or FTA® paper (Whatman, Inc., Newton MA) or Generation® Capture Cards (Gentra Systems, Minneapolis MN) or polyester material, *e.g.*, Generation® Capture Columns (Gentra Systems, Minneapolis MN). Storage media and use of such media are disclosed, *e.g.*, in WO 00/39010 and US 6,294,203; 6,447,804; 6,291,179; 6,322,983; 5,939,259; 6,168,922; and 6,410,725. such storage media may include one or more "elements" of an archive and analysis system. Storage media may include solid state storage media or an "element" in a GenVault storage system wherein an element containing a nucleic acid-containing sample may be manipulated for archiving, accessing, and analysis of the sample, *e.g.*, as disclosed in U.S. Patent Application Serial Nos. 10/005,415; 10/005,529; 10/007,355; 10/150,770; 10/150,771; 10/252,362; and 10/303,647. Storage media can optionally contain a detergent, *e.g.*, SDS, which may serve as a stabilizer, a preservative, and/or a denaturing and lysis agent, or the media may contain a chaotropic agent. It is understood that FTA® paper is chemically treated in a way that allows for the rapid isolation of high-purity DNA, because when samples are applied to FTA®-treated paper, cell lysis occurs and high molecular weight DNA is immobilized within the paper matrix. FTA® may be used as storage media in the methods of the present invention. In another embodiment, Gentra Generation® media, in card or column form, is used as storage medium in the methods of the present invention.

"Nucleic acid" or "polynucleotide" refers to purine- and pyrimidine-containing polymers of any length, either polyribonucleotides or polydeoxyribonucleotides or mixed polyribopolydeoxyribo nucleotides. This includes single- and double-stranded molecules, i.e., DNA-DNA, DNA-RNA and RNA-RNA hybrids, as well as "protein nucleic acids" (PNA) formed by conjugating bases to an amino acid backbone. This also includes nucleic acids containing modified bases.

DNA refers to deoxyribonucleic acid in the form of linearly linked nucleotides containing the sugar deoxyribose, phosphate groups, the bases adenine (A), thymine (T), guanine (G) and cytosine (C), and optionally containing modified bases. DNA bases may be modified by, *e.g.*, alkylation (*e.g.*, methylation) or deamination, generating modified bases such as N-6-hydroxylaminopurine (HAP), 5-methylcytosine, formamidopyrimidines, 8-hydroxyguanine, and 5,6 hydrated thymines. DNA includes single-stranded DNA (ssDNA), double-stranded DNA (dsDNA), triple-stranded DNA, zDNA, and other forms of DNA

RNA refers to ribonucleic acid in the form of linearly linked nucleosides containing the sugar ribose, phosphate groups, the bases adenine (A), cytosine (C), guanine (G) and uracil (U) and optionally containing modified bases. Classes of RNA molecules include messenger RNA (mRNA), transfer RNA (tRNA), ribosomal RNA (rRNA), and other small RNAs such as small nuclear RNA (snRNA) involved in mRNA splicing, small nucleolar RNA (snoRNA) involved in modification of ribosomal RNAs, and micro-RNA (miRNA) that regulate gene expression, including small interfering (siRNA) and small temporally regulated RNA (stRNA). RNA bases can by modified by, *e.g.*, generating modified bases such as N2,2,7, tri-methylguanosine (m3G), 2'-0-methyladenosine (A3), 2'-0-methylcytosine (C3), 2'-0-methylguanosine (G3), 2'-0-methyluridine (U3), pseudouridine (F), N6-methyladenosine (A6), 2-methylouanosine (G2).

### Methods and kits for eluting nucleic acids

The present invention provides a simple but elegant method for eluting dried nucleic acids, especially DNA, from storage media. In accordance with the invention, nucleic acids are eluted by using an alkaline elution buffer, wherein the nucleic acid-containing sample on storage media soaks in a buffer having a pH value between pH 11 to pH 12.7 In one embodiment, an elution buffer of the present invention has a pH value of about 11.4., In one embodiment, an elution buffer of the present invention.has a pH value of about 11.5. In one embodiment, an elution buffer of the present invention has a pH value of about 11.6. In one embodiment, an elution buffer of the present invention has a pH value of about 11.7. In one embodiment, an elution buffer of the present invention has a pH value of about 11.8.

It should be noted that the present disclosure does not provide a sharp pH optimum for optimal elution. Rather, it has been noted that a desirable efficiency of elution is achieved using buffers having pH values between pH 11 and pH 12. In contrast, little nucleic acid is eluted when the pH of the elution buffer is about or below pH 9.0. Differences between the elution efficiency of elution buffers within the range of about pH 11 to about pH 12 may or may not be statistically significant. Replying on the present disclosure, one of skill in the art can select the desired pH of the elution buffer for a particular experiment based on a variety of factors including, but not limited to, the form and type of nucleic acid to be eluted, the nature and quality of the sample, the properties of the storage media, the choice of buffer, the properties of the buffer, and the intended downstream use.

Suitable buffering agents for practicing the present invention include, but are not limited to, organic buffering agents such as CABS (4-(cyclohexylamino) -1-butanesulfonic acid), CAPS (3-(cyclohexylamino)-1-propanesulfonie acid), CAPSO (3-(cyclohexylamino-2-hydroxy-1-propanesulfonic acid), CHES (2-(cyclohexylamino) ethanesulfonic acid), EPPS (N-(2-hydroxyethyl)piperazine-N'-(3-propanesulfonic acid)), HEPES (N-(2-hydroxyethyl)piperazine-N'-(2-ethanesulfonic acid)), MES (2-(N-morpholino) ethanesulfonic acid), MOPS ((3-(N-morpholino) propanesulfonic acid), PIPES (piperazine-N,N'-bis (2-ethanesulfonic acid)), TAPS ([(2-hydroxy-1,1-bis [bydroxymethyl]ethyl) amino]-1-propanesulfonic acid), Tris (Tris (hydroxymethyl) aminomethane hydrochloride), ethanolamine, and 3-amino-1-propanesulfonic acid (all commercially available *e.g.*, from Sigma Aldrich or other suppliers). Inorganic buffering agents such as sodium phosphate and potassium phosphate are also suitable for practicing the methods provided herein. Elution buffers of the present invention can include combinations of more than one buffering agent. It has been observed that buffers having alkylsulfonate moiety connected through a secondary amine linkage appear to be particularly suitable for practicing the methods disclosed herein. Without wishing to be limited by this theory, it is proposed that such buffers may fit into the DNA minor groove, which could contribute to destabilizing the bound DNA and solubilizing the molecule. It has further been observed that certain buffers, *e.g.*, Tris and ethanolamine, perform well at pH values wherein the buffer is not charged, which leads to the conclusion that buffers need not be in a charged state to be suitable for use in the methods of the present invention. One of skill in the art can identify other buffers suitable for use in the methods of the present invention.

Elution buffers of the present invention include buffering agents at a concentration sufficient to achieve a desirable elution efficiency. One of skill in the art can determine a suitable concentration of each buffering agent depending on factors including buffer strength, pKa, solubility, and cost. Elution buffers of the present invention can include buffering agents at concentrations between 1 and 100 mM, preferably between 1 and 1 and 20 mM, more preferably between 5 and 15 mM. Elution buffers of the present invention can include one or more buffering agents at 1 mM, 2 mM, 5 mM, 10 mM, 15 mM, 20mM, 25 mM, 30 mM, 40 mM, or 50 mM.

In one embodiment, an elution buffer of the present invention includes 10 mM Tris at a pH from about pH 11 to about pH 12. In one embodiment, an elution buffer of the present invention includes 10 mM Tris at a pH from about pH 11.4 to about pH 11.8. In one embodiment, an elution buffer of the present invention includes 10 mM Tris at a pH of about pH 11.0. In one embodiment, an elution buffer of the present invention includes 10 mM Tris at a pH of about pH 11.1. In one embodiment, an elution buffer of the present invention includes 10 mM Tris at a pH of about 11.2. In one embodiment, an elution buffer of the present invention includes 10 mM Tris at a pH of about 11.3. In one embodiment, an elution buffer of the present invention includes 10 mM Tris at a pH of about 11.4. In one embodiment, an elution buffer of the present invention includes 10 mM Tris at a pH of about 11.5. In one embodiment, an elution buffer of the present invention includes 10 mM Tris at a pH of about 11.6. In one embodiment, an elution buffer of the present invention includes 10 mM Tris at a pH of about 11.7. In one embodiment, an elution buffer of the present invention includes 10 mM Tris at a pH of about 11.8. In the exemplary embodiment disclosed in Example 2 (Table 1), DNA is eluted from samples of human blood dried on FTA® elements using any of the following elution buffers: 10 mM Tris at about pH 11.4, 10 mM Tris at about pH 11.5 (11.51), 10 mM Tris at about pH 11.6 (11.62), 10 mM Tris at about pH 11.7 (11.72) and 10 mM Tris at about pH 11.8 (11.81)

In one embodiment, an elution buffer of the present invention includes any of the following at a concentration of about 10 mM, having a pH of between about pH 11 and about pH 12: CABS, CAPS, CAPSO, CHES, EPPS, HEPES, MES, MOPS, PIPES, TAPS, Tris, Ethanolamine, 3-amino-1-propanesulfonic acid, sodium phosphate, or potassium phosphate. Elution buffers of the present invention can include more than one buffering agent. In one embodiment, an elution buffer of the present invention includes any of the following at a concentration of about 10 mM, at a pH of about 11.4: CABS, CAPS, CAPSO, CHES, EPPS, HEPES, MES, MOPS, PIPES, TAPS, Tris, ethanolamine, 3-amino-1-propanesulfonic acid, sodium phosphate, or potassium phosphate. In one embodiment, an elution buffer of the present invention includes any of the following at a concentration of about 10 mM, at a pH of about 11.6: CABS, CAPS, CAPSO, CHES, EPPS, HEPES, MES, MOPS, PIPES, TAPS, Tris, ethanolamine, 3-amino-1-propanesulfonic acid, sodium phosphate, or potassium phosphate. In one embodiment, an elution buffer of the present invention includes any of the following at a concentration of about 10 mM, at a pH of about 11.8: CABS, CAPS, CAPSO, CHES, EPPS, HEPES, MES, MOPS, PIPES, TAPS, Tris, ethanolamine, 3-amino-1-propanesulfonic acid, sodium phosphate, or potassium phosphate. In a particular embodiment, an elution buffer includes 10 mM CABS at a pH of about 11.8. In a particular embodiment, an elution buffer includes 10 mM CAPS at a pH of about 11.8. In a particular embodiment, an elution buffer includes 10 mM CHES at a pH of about 11.8. In a particular embodiment, an elution buffer includes 10 mM 3-amino-1-propanesulfonic acid at a pH of about 11.6. It is understood that, in accordance with the methods of the invention provided herein, it is possible to use elution buffers containing buffers such as CABS (4-(cyclohexylamino) -1-butanesulfonic acid), which effectively elutes DNA from dry storage media, but would normally interfere with other DNA analysis methods.' After elution, the eluted nucleic acids can be separated from the elution buffer, *e.g.*, by binding to a DNA-binding material which is then washed to removed the elution buffer. By way of example, DNA-binding magnetic beads and DNA-binding columns such as those described in Examples 7, 8 and 9 can be used to separate DNA from the original elution buffer.

The present invention can be practiced using elution buffers that do not contain a chelating agent. However, chelating agents may optionally be used, depending on the particular embodiment. Optional chelating agents include, but are not limited to, EDTA or EGTA, typically in the range of about 0.1 mM to about 100 mM, more preferably in the range of about 0.5 mM to about 50 mM, and most preferably in the range of about 1 mM to about 10 mM. Chelating resins also may be used, including, but not limited to, cross-linked polystyrene beads (*e.g.*, CHELEX™), cross-linked agarose beads with tris(2-aminoethyl)amine, iminodiacetic acid, Duolite™ C-467, Duolite™ GT73, typically at a concentration in the range of about 0.01% (w/v) to about 1% (w/v), more preferably about 0.025%. (w/v) to about 0.5% (w/v), most preferably 0.05% (w/v) to about 0.2% (w/v).

Preservatives, *e.g.*, sodium azide, may optionally be used. When used, preservatives are typically used at low concentrations, in the range of about 0.1% to 0.4%. Stabilizers, *e.g.*, polyethylene glycol, may optionally be used. When used, stabilizers are typically used at concentrations in the range of about 0.04% (w/w) to about 1% (w/w).

Other optional ingredients that may be used in the elution buffers of the present invention will be apparent to those of skill in the art. In accordance with one aspect, elution buffers can include detergents. In an exemplary embodiment described in Example 5, an elution buffer containing 10 mM Tris at pH 11 and 1% SDS was used to elute nucleic acids from paraffin-embedded tissues. Optionally, elution buffers can include ingredients that are useful for further manipulation of the eluted nucleic acids. In an aspect described herein in which PCR will be performed on the eluted DNA, the elution buffer can include *Taq* polymerase buffer, nucleotide triphosphates, and primers at concentrations that will facilitate PCR amplification of the eluted DNA.

In accordance with another aspect, the method for room temperature elution of nucleic acids as provided herein includes one or more optional washing or wetting steps prior to elution. Wash steps can be carried out to remove unwanted cellular debris, proteins, or other contaminants. Wash steps can also be used to remove components of previously used reagents such as detergents or chelating agents. Wash steps also serve to wet the dry storage media and the sample dried thereof, although it is understood that sufficient wetting is accomplished by the elution buffer alone. As used herein, "wash" includes, but is not limited to, an aqueous, detergent, solvent, or enzymatic wash. A "wash solution" or "wash buffer" may include aqueous or non-aqueous solvents, or a combination of aqueous and non-aqueous solvents. Non-aqueous solvents include, but are not limited to, ethanol, acetone, phenol, chloroform, acetonitrile, dimethylsulfoxide, or any polar or nonpolar non-aqueous solvent suitable for use in accordance with the present invention. A wash solution may contain one or more additional components including, but not limited to, buffers, salts, detergents (*e.g.*, Triton, Tween, SDS, CHAPS), protein, preservatives, and stabilizers. Wash solutions may contain one or more different proteins, *e.g.*, enzymes (active enzymes) to carry out certain reactions and/or proteins for blocking or buffering the solution. Wash solutions may contain enzymes including, but not limited to, Proteinase K, RNase, DNase, kinase, or methylase. Wash solutions may contain bovine serum albumin (BSA), casein (or milk), or denatured proteins for blocking or buffering. In one embodiment, samples on storage media are incubated with a wash buffer containing 1% Triton X-100, 10 mM Tris pH 8.0 and 0.1 mM EDTA. Samples on storage media may be incubated with a wash buffer containing 1% Tween 20, 10 mM Tris pH 8.0 and 0.1 mM EDTA. Wash buffers can be removed by suitable means including, but not limited to, centrifugation, aspiration, or absorption. One of skill in the art can determine whether one or more wash steps are in accordance with a particular embodiment. If washing is selected, one of skill in the art can determine the composition of a wash solution suitable for the embodiment. It is understood that wash steps can also serve to wet the storage media prior to elution.

The method for room temperature elution of nucleic acids in accordance with the invention may include a shift in reagent pH between washing and elution steps. The reagent used for one or more washing/wetting steps may be replaced with more alkaline elution buffer. A pH shift may be achieved by making the reagent used for washing/wetting more alkaline, *e.g.* by adding concentrated NaOH or alkaline buffer. Without wishing to be limited by this theory, the alkaline pH may function to effectively neutralize the bound nucleic acid by deprotonation, such that the electrostatic interaction between the strands of nucleic acid and the storage medium is weakened and the nucleic acid can be eluted. Alternately, the alkaline pH of the elution may act by a different mechanism, as it should be noted that increasing the pH of the solution may also increase charge on nucleic acids, primarily due to ionization of G and T residues, which may alter the interaction between nucleic acids and any storage medium to which they may be bound. Without wishing to be limited by this theory, the alkaline pH may shift the adsorption equilibrium for nucleic acid binding in the direction of solution-phase solubilization. Preferably, the elution pH is selected so that it does not weaken purine-pyrimidine bonds, i.e., complimentary base pairing and salt bridges.

In accordance with the invention, double stranded DNA (dsDNA) may be eluted from blood spots, buccal cells, cells, viruses, and other DNA source material dried on storage media as defined herein above and including FTA® paper, IsoCode® paper. Briefly, one or more simple wash steps are carried out to remove unwanted cellular debris and proteins. Optional wash steps can also be used to remove components of previously used reagents such as detergents or chelating agents. In the elution step, the sample is soaked in a more alkaline elution buffer, which releases DNA from the storage media into the elution buffer. Generally, the DNA-containing eluate (DNA in elution buffer) is then separated from the storage media and optionally neutralized with equilibration buffer to stabilize the DNA for storage in solution. The DNA-containing eluate can be used directly for subsequent analysis, or DNA may be recovered and/or separated from the elution buffer, *e.g.*, by standard buffer exchange methods, by precipitation, or by binding to a DNA-binding material. The elution step may optionally be repeated one or more times, and the DNA-containing eluates can be combined to enhance yield.

The present invention provides methods for efficient room temperature elution of nucleic acids, especially DNA, from storage media. In contrast, disclosed methods for eluting nucleic acids from storage media utilize techniques that involve extreme conditions or undesirable reagents, or have other drawbacks. For example, some disclosed methods use elevated temperature to remove DNA from paper storage media such as FTA® and IsoCode®, typically producing less stable single stranded DNA (ssDNA) rather than the preferred dsDNA. For example, US 6,410,725 discloses a two-step method to extract DNA from biological samples on cellulosic materials including FTA® and from S&S 903 cellulose paper, with a first step carried out at a temperature between 40°C to 60°C and a second step at a temperature between 45° to 100°C. Other disclosed methods require organic solvents or other aggressive chemicals that pose health and environmental hazards, for example phenol, or a combination of sodium metasilicate and an ether, to remove DNA, typically as small fragments of ssDNA, from storage media. For example, US 6,503,716 discloses the use of a buffer containing sodium metasilicate and an ether, at a pH of between 7 to 10, at ambient temperature, to extract DNA from a variety of biological samples on dried storage media. Many previously disclosed methods do not lend themselves to simple automation, *e.g.*, using common liquid handler robots, due to the organic solvent content and/or the need for extensive heating. When undesirable reagents such as phenol or sodium metasilicate are used, the eluted DNA may be suitable for some uses such as PCR but it is nonetheless "contaminated" with the organic solvent and the metasilicate, and must be further purified. Publications disclosing the usefulness of DNA storage media have also noted the difficulty of removing DNA from storage media.

The invention provides methods for isolating DNA from As storage media using easy-to-prepare reagents at room temperature. Without wishing to be limited by this theory, it is believed that carrying out the steps at room temperature may minimize or ameliorate some of the potentially disruptive effects of the high pH of the elution buffer on DNA structure, including dsDNA base-pairing and/or salt bridges as noted *infra*.

Exposure of eluted nucleic acids to high pH can be minimize by an optional "quenching" step. The "quench" step may be an isolated step undertaken to lower the pH of the eluate, *e.g.,* by adding an acidifying agent, or by buffer exchange with a buffer having a pH of between about 5.0 and 10.0 pH units, preferably between about 8.0 and 9.0 pH units. The "quench" step may occur as part of a subsequent analysis wherein the pH may be adjusted, *e.g.*, by acidification, desalting, or buffer exchange as part of a protocol to achieve a desired (optimal) pH and/or buffer composition for carrying out a particular assay. When DNA-binding materials such as affinity columns or magnetic beads are used to selectively extract DNA (*see*, *e.g.*, Examples 7-9, below), the DNA is separated from the alkaline elution buffer and introduced into a different buffer suitable for the next step. Preferably, an alkaline solution containing eluted DNA is quenched prior to carrying out PCR, LCR, or RFLP.

In accordance with one aspect; the present invention provides typical yields in the range of about 50% to about 100%, preferably about 60% to about 90%, more preferably about 70% to about 80%, of the DNA present in the sample on the storage media. Yield of DNA can be quantitated using methods known in the art, including DNA stains and DNA intercalators as well as by UV spectroscopy. The invention may provide high quality DNA, in particular long pieces of high molecular weight dsDNA, that can be used without requiring any further treatment. The presence of dsDNA may be verified using PicoGreen® reagent (Molecular Probes, Inc., Eugene OR). The invention provides methods for eluting DNA suitable for subsequent analysis, *e.g.*, in amplification reactions, sequencing reactions, labeling reactions, annealing reactions, restriction digests, ligations, reverse transcriptase reactions, hybridizations, or Southern blots.

Nucleic acids eluted as provided here may be in double stranded form, or in single stranded form, or in a mixture of forms. The form of the eluted nucleic acid depends on various factors including, but not limited to, pH of the elution buffer, buffer strength, properties of the storage medium, and quality of the nucleic acid-containing sample. It has been noted that room temperature elution, using an elution buffer having a pH of about pH 10, elutes nucleic acids that are predominantly in the double stranded form, in particular dsDNA. It has further been noted that room temperature elution as provided herein, using an elution buffer having a pH of about, pH 12, elutes nucleic acids that are predominantly in the single stranded form, in particular ssDNA. Without wishing to be limited by this theory, it is proposed that elution at more alkaline pH, *e.g.*, above about pH 11.0, probably elutes nucleic acids that are predominantly in single stranded form, but that subsequent neutralization of the eluate may permit or facilitate pairing of any complementary strands to generate double-stranded forms.

Nucleic acids eluted as provided herein may be used directly in further assays and experiments without further purification. The eluate may be run directly on an agarose gel for direct analysis of the isolated nucleic acids. The eluate can be used directly, with no further manipulation, as a template for amplification. The eluate fraction can also be used as a source of template DNA for other applications such as sequencing, labelling reactions, or use as a probe or primer. The eluate can be further purified or enriched before use. The methods of the present invention are especially well-suited for preparing nucleic acid extracts for use in amplification techniques including but not limited to polymerase chain reaction (PCR), ligase chain reaction (LCR), reverse transcriptase cDNA synthesis, rtPCR, microarrays, gene chips, DNA or RNA hybridization techniques including restriction fragment length polymorphism (RFLP), and for use in any high-throughput or automated process.

PCR can be carried out on DNA in eluates obtained by the methods provided herein. In accordance with a particular aspect, PCR can be carried out on DNA in the eluates of the present invention without any further treatment (*i.e.,* PCR can be carried out directly on the DNA-containing eluate) and provides PCR products having a length of up to several kilobases (kB). As demonstrated in Examples. 1, the eluate was used directly as a source of template DNA for the PCR reaction. As illustrated in Example 1, DNA isolated according to the methods of the invention can be used directly in PCR amplification experiments.

The methods provided herein may be suitable for use as part of high throughput or automated processes. As used herein, an "automated system" includes "automatic fluid delivery systems" including hand-held and robotic fluid delivery systems. Typically, automatic fluid delivery systems are devices which dispense and remove fluid reagents to and from individual wells of multi-well reaction plates. Hand-held automatic fluid delivery systems comprise a single plunger handle with multiple fluid aspirating and dispensing ends to simultaneously aspirates and dispense fluid of a fluid reaction system from single or multiple fluid reaction vessels simultaneously. Robotic automatic fluid delivery systems are computer operated rather than hand-held and include such products as, for example, BIOMEK 2000 (Beckman Instruments, Fullerton, Calif.), Zymark Benchmate (Zymark, Hopkinton, Mass.), ROSYS PLATO (Rapperswil, Switzerland) and others.

The methods provided herein may be suitable for use as part of an automated archive and analysis system, *e.g.*, as disclosed in U.S. Patent Application Serial Nos. 10/005.415; 10/005,529; 10/007,355; 10/150,770; 10/150,771; 10/252,362; and 10/302,64 7.

The present invention also can be adapted to enrich, purify, or wash a previously extracted nucleic acid sample. For example, a nucleic acid sample can be applied to storage media, and nucleic acids may be eluted using the room temperature elution method provided herein.

Furthermore, the methods of the present invention are inexpensive, simple and quick to make or use compared to other nucleic acid extraction reagents, methods known in the art. Unlike other methods of extracting nucleic acids, the methods of the invention can be performed without a heat source. The methods of the invention can be performed rapidly, preferably between about 30 minutes to about 60 minutes. The reagents used in the methods provided herein are also environmentally benign.

The methods of the present invention can be used to optimize nucleic acid recovery, depending on the sample, the storage media, and the intended use. When a high nucleic acid concentration in the eluate is desired, the methods of the invention can be adapted to extract most of not all of the nucleic acids in a minimal eluate volume. Generally, an eluate nucleic acid concentration of about 1 ng/ml or greater is considered desirable, as a solution with this concentration is often suitable for further use without the need for additional concentration. The methods of the present invention may produce eluates having a DNA concentration of about 1 ng DNA/ml or higher. The present invention may provide methods for producing eluates having about 10 ng/ml, about 50 ng/ml, about 100 ng/ml, about 250 ng/ml, about 500 ng/ml, about 1000 ng/ml, about 1500 ng/ml, about 2000 ng/ml, about 2500 ng/ml, about 3000 ng/ml, about 3500 ng/ml, about 4000 ng/ml, about 4500 ng/ml, about 5000 ng/ml, about 5500 ng/ml, about 6000 ng/ml, about 6500 ng/ml, about 7000 ng/ml, about 8000 ng/ml, about 9000 ng/ml, or about 1 mg/ml DNA or RNA.

### Nucleic acid-containing samples

As detailed above, the method of the invention elutes nucleic acids from a nucleic acid-containing sample on storage media. The invention provides methods that can be used to isolate nucleic acids from virtually any nucleic-acid-containing sample on storage media as defined above. Methods of the invention are suitable for isolating DNA or RNA from samples.

In accordance with one aspect, the sample from which DNA is eluted as provided herein can contain DNA molecules in any form. The DNA molecule can be, for example, genomic DNA. Genomic DNA can be isolated using the methods of the invention from any source, including an entire chromosome or any part of a chromosome. Genomic DNA can be mutant, wild-type, or synthetic. Genomic DNA can comprise DNA from another source (*e.g.*, a gene from another organism) introduced into the genomic DNA by any technique known in the art. Genomic DNA can be found in yeast artificial chromosomes (YACs), bacterial artificial chromosomes (BACs) or other vectors, and may optionally be introduced into a heterologous organism. Genomic DNA can comprise a coding sequence or a non-coding sequence or part of a coding sequence or open reading frame (ORF). The coding sequence can be wild-type or mutant, full-length or truncated. The non-coding sequence can be, by way of nonlimiting example, a centromere, a telomere, an intergenic region, an intron, a transposon, a microsatellite sequence, mitochondrial DNA, chloroplast DNA, or other organellar DNA.

DNA in a sample may further include, or may consist of, plasmid DNA molecule from any source or organism. The term "plasmid DNA" refers to all DNA molecules within a cell that are not part of the cell's normal complement of chromosomes, such that plasmid DNA includes artificial chromosomes, extrachromosomal DNA and organellar DNA, a naturally occurring plasmid, or a genetically engineered plasmid. DNA in a sample may be a cDNA made by reverse transcription of an RNA template, or by replication of a cDNA. DNA in a sample may be one or more DNA oligonucleotides of any length.

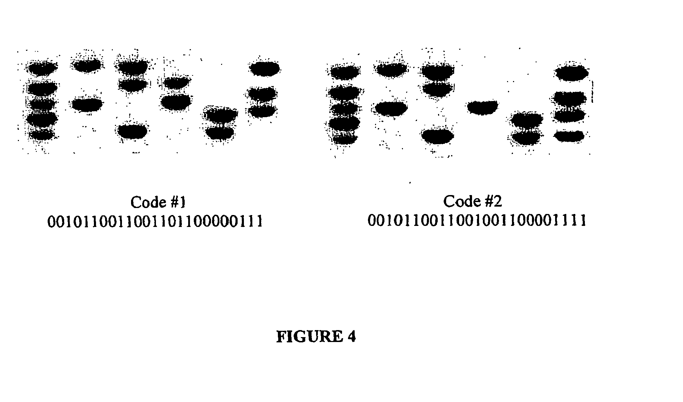

A sample on storage media, optionally contained in a sample carrier, may contain additional DNA molecules not originally found in the sample. Additional DNA molecules may be added to a sample prior to applying the sample to storage media, to provide means for identification or tracking of the sample, or to provide quality control measurements. Alternatively, a sample may be added to storage media already containing the additional DNA molecules which provide means for identification or tracking of the sample, or to provide quality control measurements. Additional DNA molecules can be small, *e.g*., oligonucleotides.. An additional DNA molecule can be a "biological bar code" such as a GenVault GenCode molecule used to identify a sample, *e.g*., as shown in Figure 4 and disclosed in Example 6.

A sample on storage media may contain RNA. Methods of the invention are suitable for eluting RNA from one or more samples. In accordance with one aspect, the sample from which RNA is eluted as provided herein can contain RNA molecules in any form. The RNA molecule can be, for example, include messenger RNA (mRNA), transfer RNA (tRNA), ribosomal RNA (rRNA), and small RNAs such as small nuclear RNA (snRNA), small nucleolar RNA (snoRNA), and micro-RNA (miRNA) including small interfering (siRNA) and small temporally regulated RNA (stRNA). A sample on storage media may contain viral RNA.

The source of the nucleic acid can be any cell or virus, or any other composition, housing or structure containing nucleic acid. The nucleic acid can be extracted from any kind of cell, including both prokaryotic and eukaryotic cells. A sample may include any type of eukaryotic cell, and may include one cell or a collection of cells. The methods provided herein may be sufficiently powerful to elute nucleic acids from a single cell, providing material for subsequence analysis such as PCR. A sample may include a collection of cells, including cells from unicellular eukaryotes, multicellular eukaryotes, or a sample of cells (*e.g,* a tissue sample) from a multicellular eukaryote. Any type of prokaryotic cell can be used, including eubacteria and archaebacteria, and gram-positive and gram-negative bacteria. The prokaryote can be a pathogenic bacterium. The eukaryotic cell can be, for example, a pathogenic eukaryotic microorganism, a blood cell or a tissue cell.

A sample may include material from a single organism, a single species, a plurality of organisms, and/or a plurality of species. A sample may consist of a single unicellular or multicellular organism. A sample may consist of a tissue sample taken from a single individual, *e.g.*, a human patient. A sample may contain a plurality of identical or nearly identical unicellular or multicellular organisms, *e.g.*, a sample taken from a single bacterial colony on a culture dish, or a pure algal culture. A sample may contain a plurality of identical cells derived from a single source or a single organism, *e.g.,* a sample taken from a mammalian cell culture. A sample may contain material from a plurality of organisms, *e.g.,* a environmental sample containing cells from many organisms. A sample may contain material from a plurality of cell types within a single organism, *e.g.,* a tissue sample having multiple cell types present, wherein such a sample will contain a single species of genomic DNA, but will contain a plurality of cell-specific RNAs. One of skill in the art can determine how to carry out subsequent analysis of nucleic acids eluted from a sample depending on factors including the source of the sample, the composition of the sample, and the type of analysis to be carried out.

The source of the nucleic acid can be part of a larger sample preserved on storage media. The sample may be contained in a sample carrier that includes storage media. For example, the sample can be a food sample, a clinical sample, a forensic sample, an agricultural sample or an environmental sample on storage media. The methods of the invention are effective at separating the nucleic acid from these other substances. The sample may contain, in addition to the source of the nucleic acid, additional solid or liquid. The solid can be water soluble or water insoluble. For example, the methods of the invention can be used to detect the presence of a food pathogen in a food sample on storage media. This can be done by extracting genomic DNA from the food sample using the methods of the invention and amplifying the DNA using primers specific for the pathogen.

Alternatively, the sample can contain any substance derived from an animal subject and preserved on storage media, including but not limited to blood, cerebral spinal fluid, hair, fur, saliva, sputum, semen, urine, stool, mucous, skin, a benign or malignant tumor or growth, biopsied tissue or any other type of tissue sample used in diagnosing a disease or condition. The subject can be any kind of animal, for example, a human. Alternatively, the sample can contain any substance derived from a plant subject, for example, leaf, stem, stalk, pollen, root, branch, flower, seed, bulb, spore or other plant material.

The nucleic acid extracted from the sample can be that of the subject from which the sample was obtained. It can be used, for example, to determine whether the subject has a disease or medical condition, or to determine the subject's genotype, or to determine whether a certain gene is being expressed in the subject's tissue in the sample. Alternatively, the extracted nucleic acid can be that of a pathogen or other organism in the sample. The pathogen or organism can be, for example, a bacterium, virus (*e.g.,* HIV), worm, insect or fungus. The nucleic acid can then be amplified using primers specific for the pathogen or other organism to detect the presence of the pathogen or other organism in the sample. Thus, the methods of the invention are useful in a wide variety of medical, clinical, forensic and agricultural applications.

Alternatively, the sample can comprise, for example, soil, dirt, landfill, garbage or waste, plant or animal matter, water (including, *e.g.,* fresh water, salt water or waste water) or a sample collected from a structure (*e.g.,* a building) or a device (*e.g.,* an air conditioner) and preserved on storage media. The extracted nucleic acid can be diagnostic of the presence of a microorganism in the sample. The microorganism can be pathogenic or otherwise harmful to another species, for example, to humans. The presence of the microorganism in the sample can be used to diagnose, assess, monitor or remedy environmental damage to the source of the sample (*e.g.*, a deficiency or imbalance of nutrients or the presence of a toxin).

Although the method of the invention has been exemplified in terms of isolating a nucleic acid from a biological or other sample comprising a cell or virus preserved on storage media, those of skill in the art will appreciate that the methods and reagents may be used to isolate and/or purify nucleic acids from virtually any source. For example, the methods and reagents may be used to isolate nucleic acids from acellular sources such as *in vitro* reactions preserved on storage media, such as *in vitro* transcription or reverse transcription reactions.

### Uses of eluted nucleic acids

The methods disclosed herein can be used to elute nucleic acids from a variety of DNA-or RNA-containing samples preserved on media intended for storage as defined herein above.

The methods are not limited to samples that are archived, but include a general method for nucleic acid extraction and recovery. In particular, the methods of the present invention provide high quality DNA that can be used in a large number and wide variety of applications, some of which are described in Sambrook et al. (eds.), 1989, Molecular Cloning: A Laboratory Manual, Cold Spring Harbor Press, N.Y. and Ausubel et al. (eds.), 2000, Current Protocols in Molecular Biology, John Wiley & Sons, N.Y.

The present invention provides methods for eluting nucleic acids from samples on storage media, thereby providing nucleic acids for subsequent analysis. As used herein, "subsequent analysis" includes any analysis which may be performed on nucleic acids eluted from a sample on storage media. Because analysis of eluted nucleic acids provides information concerning the sample from which the nucleic acids were eluted, the term "subsequent analysis" necessarily refers to analysis of the nucleic-acid-containing samples itself. The sample on storage media may be analyzed *in situ,* wherein the nucleic acids are made available for analysis but not removed from the storage media, *i.e.,* elution buffer is added to a sample on storage media, and regents for subsequent analysis (*e.g.* colorimetric reagents) are then added to the same tube. The nucleic-acid-containing sample may first be removed from the dry solid medium prior to analysis.

The nucleic-acid-containing sample may also be subjected to chemical, biochemical or biological analysis. Examples of subsequent analysis which may be performed on samples of nucleic-acid-containing sample stored on the dry solid medium include purification, polymerase chain reaction (PCR), ligase chain reaction (LCR), reverse transcriptase initiated PCR, DNA or RNA hybridization techniques including restriction fragment length polymorphism (RFLP) and other techniques using genetic or DNA or RNA probes, genomic sequencing, whole genome amplification, STR, and SNP analysis.

As demonstrated in Example 1, the eluate recovered from room temperature elution of a blood sample on storage media may be run directly on an agarose gel for direct analysis of the isolated nucleic acids, and can be used for amplification. The eluate fraction can also be used as a source of template DNA for other applications such as sequencing, labelling reactions, or use as a probe or primer. The methods of the present invention are especially well-suited for preparing nucleic acid extracts for use in amplification techniques including but not limited to polymerase chain reaction (PCR), ligase chain reaction (LCR), reverse transcriptase cDNA synthesis, rtPCR, microarrays, gene chips, DNA or RNA hybridization techniques including restriction fragment length polymorphism (RFLP), and for use in any high-throughput or automated process.

Nucleic acids eluted by the methods provided herein can be enriched or purified prior to subsequent analysis. Purification can refer to separating nucleic acid molecules of a certain type, *e.g.*, DNA, away from other components in the eluate. Purification can also refer to separating or fractionating molecules in a sample, in order to enrich for nucleic acids having certain desired characteristics such as a particular size, conformation, or sequence. The methods provided herein can be adapted to include one or more additional steps to separate a particular nucleic acid component from other nucleic acids or from other components. Likewise, the methods provided herein can be adapted to include one or more additional steps to selectively enrich for a particular nucleic acid component.

When, for example, using planar chromatography, eluted nucleic acids can be run on an agarose or polyacrylamide gels to separate the nucleic acids by size or topology. In other embodiments, RNA or DNA can be removed by contacting the isolated nucleic acids with RNAse or DNAse, respectively. Alternatively, the wash buffer or elution buffer may include an RNAse or DNAse if desired, provided that the presence of enzymes is compatible with the filtration or extraction system used, and provided that the pH of the solution does not denature or otherwise deleteriously affect the activity of the enzyme.

Nucleic acids can be separated or purified using affinity chromatography, *e.g*., Sephadex or polyA columns. As shown in Examples 7, 8, and 9 below, DNA can be purified from the eluate obtained by room temperature elution of human blood from FTA® elements (storage media), where the DNA can be purified using commercially available technologies including DNA-binding magnetic beads (Examples 7, 8), phenol:chloroform extraction of eluates (Example 9), or DNA-binding chromatography-based products such as columns available from Qiagen and Gentra Corporation. A hybridization step can be added to separate nucleic acids according to their sequences, *e.g.* using sequence-specific probes linked to "tags" by which the probes, and any nucleic acids hybridized thereto, can be recovered. These, and other, methods for purification, separation, or fractionation of nucleic acids are known to those of skill in the art.

The methods of the present invention can be used to isolate virtually any type of nucleic acid, regardless of its length or sequence, single or double stranded, from storage media as defined above. The operator need not know the sequence of the nucleic acid to be extracted. The nucleic acid also can comprise a non-nucleic acid component. For example, the nucleic acid can be covalently modified (*e.g.,* with biotin, avidin, streptavidin) or otherwise labeled (*e.g.,* with a radioactive isotope, fluorescent marker, molecular beacon, conjugated nucleotide, or protein tag). One of skill in the art can follow the teachings provided herein to determine the suitability of the present invention for eluting non-traditional nucleotides, chemically modified nucleotides, artificial nucleotides, synthetic derivatives such as PNAs, nucleotide substitutes, and classes of DNA such as z-DNA. One of skill in the art can modify experimental conditions within the scope of the present disclosure, in order to obtain desired results using the present invention to elute the nucleic acid present in a sample.

Optionally, proteins associated with the nucleic-acid-containing sample can be denatured for eliminated. Optionally, the sample can be incubated with enzymes including, but not limited to, proteinase K, lysozyme, papain, or other proteases, prior to elution of nucleic acids from the sample. FTA® elements with samples dried thereon may be incubated with proteinase K in a suitable buffer, prior to elution of nucleic acids from the sample. Samples can also be enzymatically treated to remove non-protein contaminants including, but not limited to, lipids, lipopolysaccharides, pigments, chitin, or cellulose (especially if sample is on non-cellulosic storage media). However, enzymatic (*e.g.,* protease) treatment is not required to practice the methods provided herein. In fact, the pH shift provided in certain embodiments, wherein the sample is washed at a lower pH and eluted at using a high pH reagent, may be sufficient to denature potentially interfering proteins in a sample. Alternatively, the storage media may be made of a material to which nucleic acid binds and proteins do not bind, with the result that there is little interfering protein present in the sample being eluted. Alternatively, the storage media may contain, chaotropic agents or specific protein inhibitors. Alternatively, proteins associated with the sample are optionally removed from the sample stored on dry solid medium prior to analysis by PCR, LCR, RFLP, reverse transcriptase initiated PCR, genetic probing or other technique.

In addition, dried samples on storage media can be analyzed using a variety of diagnostic procedures that are often performed on fresh samples, including but not limited to antibody tests to detect certain proteins, enzymatic assays to detect the presence and/or measure the activity of certain enzymes, tests to determine the presence of certain cell types, or tests for components such as sugars, lipids, metals, starch, or any component of interest in a particular embodiment. Dried blood samples can be analyzed using diagnostic procedures that are often performed on whole blood samples including, but not limited to, the Guthrie test for phenyl-ketonuria (PKU) enzymatic tests, *e.g.* for galactosemia, blood typing, HLA typing, cross-matching, or testing for HIV, CMV, or hepatitis.

### Kits

Also described herein are kits for eluting nucleic acids. In particular, kits for eluting DNA and/or RNA. kits may contain an elution buffer as described above. It is understood that kits also described herein necessarily also contain instructions for eluting nucleic acids in accordance with the methods of the present invention. kits may further contain, *inter alia,* wash buffer, neutralization buffer, and storage media for eluted nucleic acids. Kits may further contain enzymes, *e.g.*, proteinase K.

Furthermore described herein are kits useful for recovering eluted nucleic acids. Kits may contain an elution buffer, an optional wash solution, and one or more other additional components useful for recovery of the eluted nucleic acids. Also described herein are kits useful for analyzing eluted nucleic acids, in particular DNA. Kits may contain a an elution buffer, an optional wash buffer, and one or more other additional components useful for analysis of the eluted nucleic acids. A kit may contain an elution buffer, an optional wash buffer, and one or more reagents useful for amplifying a nucleic acid of interest, including but not limited to, one or more amplification primers, one or more dioxy nucleotide triphosphates *(e.g.,* a mixture of dATP, dGTP, dCTP and/or dUTP or dTTP)one or more polymerizing enzymes (*e.g.*, DNA polymerase), *etc.* Alternatively, the kit may include one or more additional reagents useful for sequencing a nucleic acid of interest, *e.g.,* one or more sequencing primers (labelled or unlabelled, or covalently modified), one or more deoxynucleotide triphosphates (*e.g.,* a mixture of dATP, dGTP, dCTP and dUTP or dTTP), one or more labelled or unlabelled dideoxynucleotide triphosphate terminators (*e.g.,* ddATP, ddGTP, ddCTP and ddUTP or ddTTP) or one or more polymerizing enzymes (*e.g.,* DNA polymerase, *Taq* polymerase, *Pfu,* elongase). Furthermore, the kit may include one or more reagents useful for labelling an isolated nucleic acid, *e.g.,* one or more labelled deoxynucleotide triphosphates, one or more polymerizing enzymes, or one or more labelled or unlabelled primers.

The kits also described herein may include materials (equipment) for elution of nucleic acids from samples including, *inter alia,* holders for elements with samples dried thereon, tools for manipulating elements for nucleic acid elution, and/or vessels for collecting eluted nucleic acids. Kits may further include materials for purifying nucleic acids, *e.g.,* columns or cartridges for DNA or RNA purification from a solution, affinity media such as beads for DNA or RNA purification from a solution, or chromatographic media for purification or separation of nucleic acids. Materials for subsequent purification of eluted nucleic acids include, but are not limited to, magnetic beads for DNA purification such as those described in Examples 7 and 8, and DNA , purification columns such as those described in Example 9.

The following examples are presented for purposes of clarity and understanding and are, wherefore, to be construed as merely illustrating and not limiting the scope of the invention which is defined in the appended claims.

### EXAMPLES

### Example 1: Room temperature elution using the Beckman Biomek 2000 Liquid Handler_{.}

Eight FTA® elements in Gen Vault "mother plates" were spotted with 10 µl of whole blood, and were dried and stored. The eight DNA-containing elements were then punched out of the mother plates into the first row of wells in a 96 well filter plate (Whatman 7700-2804). Using an eight-tip head on a Beckman 2000 Liquid Handler, 800 µl of GenVault Wash Buffer #1 (also called GenVault purification reagent) was dispensed into each well containing a GenVault element. Under experimental conditions, wash buffer containing 1% Triton X-100, 10 mM Tris pH 8.0 and 0.1 mM EDTA and wash buffer containing 1 % Tween 20 with 10 mM Tris pH 8.0 and 0.1 mM EDTA, were equally effective. Each element was incubated in Wash Buffer #1 for five minutes and subsequently centrifuged for one (1) minute at 800 x g or 3,000 rpm to removed spent buffer. The process was repeated once using GenVault Wash Buffer #1, followed by three washes with Genvault Wash Buffer #2 (10 mM Tris pH 8.0, 0.1 mM EDTA) at similar volumes.

The DNA-containing elements, still in the filter plate, were then treated with 150 µl of GenVault elution buffer (10 mM Tris, pH 11.6) for ten minutes with gentle agitation on an orbital shaker. Filter plates were centrifuged for one (1) minute at 10,000 x g to recover DNA. Filtrate (eluate) was neutralized with GenVault equilibration buffer and the DNA was quantitated. DNA yields for the protocol using automated washes of elements in filter plates were equivalent to DNA yields from "standard" protocol of individual washes, where each element is washed in a well of a filter plate or in a microfuge tube, and each element is then centrifuged in an individual spin column to separate the element from the eluate. Using the same blood lot, the individual wash protocol yielded 120ng +/-15 ng (5 samples) and the automated wash yields were 109ng +/-17ng (20 samples taken over various regions of the plate). PCR of the eluted DNA showed no detectable difference in quality or sequence between the samples.

PCR was carried out on eluted DNA to detect (amplify) the following sequences: YR1 (110 pb product); 558 bp product; beta globin (1000 bp product); and 1764 bp product. PCR conditions were as follows: 16mM (NH₄)₂SO₄, 67 mM Tris-HCl (pH 8.8 at 25C), 0.01 % Tween 20,1.5mM MgCl₂, 200µM of each dNTP (dATP, dGTP, dCTP, dTTP) (Bioline, Randolph, MA), 0.2µM of each primer, 5% DMSO, and 2.5 units of Biolase (Bioline, Randolph, MA). PCR cycling conditions were as follows: 93°C for 2 minutes, 57°C for I minute, 72°C for 2 minutes, followed by 29 cycles of: 93°C for 30 seconds, 57°C for 30 seconds, and 72°C for 2 minutes. The primer sequences were:

| | | |
|---|---|---|
| YR1 (110bp product): | | |
| 5' primer: | 5' TGG GCT GGA ATG GAA AGG AAT GCA AAC 3' | (SEQ ID NO: 1) |
| 3' primer: | 5' TCC ATT CGA TTC CAT TTT TT7 CGA GAA3' | (SEQ ID NO: 2) |
| 558 bp product: | | |
| 5' primer: | 5' AGA TGA AGA ATG TGT GTG ATG GAT GTA 3' | (SEQ ID NO: 3) |
| 3' primer: | 5' GGG CTC GTA ACC ATA GGA AGG GTA 3' | (SEQ ID NO: 4) |
| | | |
| Beta Globin (1000bp product): | | |
| 5' primer: | 5' TGG TAG CTG GAT TGT AGC TG 3' | (SEQ ID NO: 5) |
| 3' primer: | 5' ATT TTC CCA CCC TTA GGC TG 3' | (SEQ ID NO: 6) |
| | | |
| 1764 bp product: | | |
| 5' primer: | 5' CAA GAA GGA GTG TCA GGG CCG GA 3' | (SEQ ID NO: 7) |
| 3' primer: | 5' CAG AGA GGT AGC CAG TCC TGT GGT G 3' | (SEQ ID NO: 8) |

### Example 2: Determination of pH optimum

The optimum pH for sample elution under certain conditions was determined. Briefly, five (5) FTA® elements were stored on and retrieved from GenVault plates as described above. Ten (10) µl of human blood was spotted on each FTA® element and allowed to dry. To test the effectiveness of elution at different pH values; with Tris as the test buffer, elements were eluted with 10 mM Tris adjusted to various pH values from 11.4 to 11.8, in increments of 0.1 pH unit.

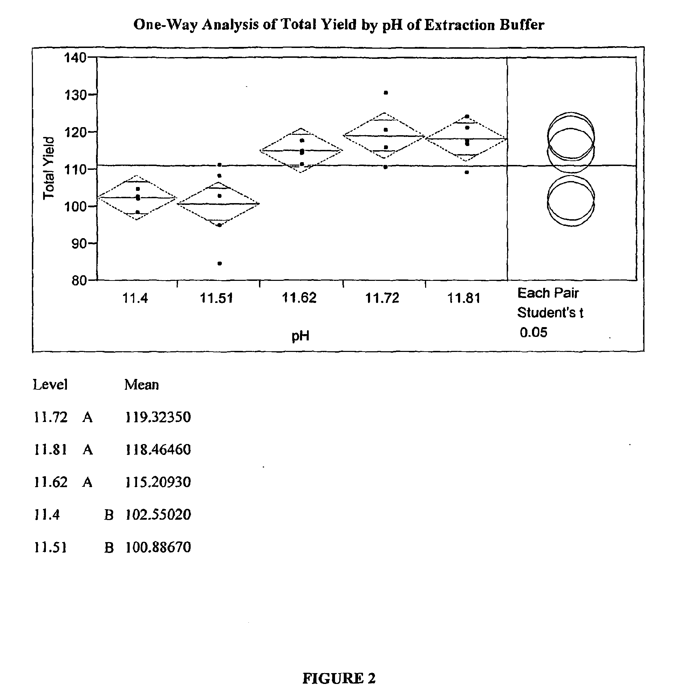

Elements containing samples were washed using the standard wash protocol (two 5-minute washes with 1% Triton x-100, 10 mM Tris pH 8.0 and 0.1 mM EDTA, followed by three 5-minute washes with 10 mM Tris pH 8.0, 0.1 mM EDTA, as described above in Example 1, above). Elements were then incubated with 150µl of elution buffer at a specific pH for 10 minutes under gentle agitation on an orbital shaker. Yields from each reaction were quantitated using PicoGreen® reagent (Molecular Probes, Eugene OR), read on a Tecan GENios plate reader, and the results were analyzed using a paired Student's T test at p = 0.05. The experimental design was developed using JMP software (a division of SAS), which is widely used for experimental design and analysis using ANOVA. Table 1 and Figure 2 show DNA yields at each pH.

**Table 1. DNA yields at each pH**

| **pH of Tris elution buffer** | **Mean DNA yield, ng** | **Significance** |
|---|---|---|
| 11.72 | 119.32350 | A |
| 11.81 | 118.46460 | A |
| 11.62 | 115.20930 | A |
| 11.4 | 102.55020 | B |
| 11.51 | 100.88670 | B |

In Table 1, values not assigned the same letter are significantly different. The highest yields were obtained using elution buffers at pH 11.72, 11.81, and 11.62, which produced yields that were not statistically different. Elution buffers at pH 11.4 and 11.51 produced yields that were statistically different from yields using higher pH buffers.

### Example 3: Effect of buffer choice

Room temperature elution of DNA from FTA® elements with 10 µl of human blood spotted on each element was carried out as described above, using a variety of buffers adjusted to different pH values.

In one experiment, elution was carried out using 10 mM monobasic potassium phosphate buffer, pH 11.2, using the elution method described in Example 1, above. The combined yield of 2 elution steps using a total of 150 µl of elution buffer produced an average yield of 20 ± 5 ng of DNA, which was about 25% of the yield obtained from identical 10 µl human blood samples in elution experiments using 10 mM Tris elution buffer at pH 11.6.

In another experiment, a series of elution reactions were carried out using 10 µl human blood samples spotted on GTA® elements, using 10 mM solutions of different buffers adjusted to different pH values. Briefly, the "standard" protocol was followed, with two (2) 5-minute washes using GenVault Wash Buffer #1 (1% Triton X-100, 1 mM Tris pH 8.0, 0.1 mM EDTA), followed by three (3) washes with GenVault Wash Buffer #2 (10 mM Tris pH 8.0, 0.1 mM EDTA), after which each element was submerged in 150 µl of elution buffer (here, buffer and pH varied) and placed on an orbital shaker with moderate agitation for 10 minutes. Finally, the elution buffer containing DNA was centrifuged through a spin column to separate eluate from the FTA® element. The entire process was then repeated.

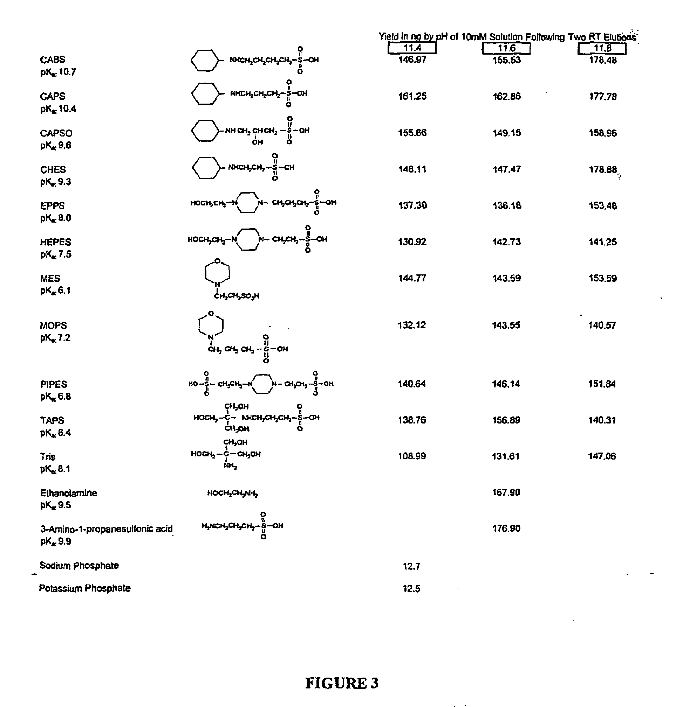

Ten mM (10 mM) solutions of CABS, CAPS, CAPSO, CHES, EPPS, HEPES, MES, MOPS, PIPES, TAPS, Tris, Ethanolamine, 3-amino-1-propanesulfonic acid, sodium phosphate, and potassium phosphate were tested. Figure 3 shows the pKₐ and structure of each buffer, and presents the average yield of two elutions carried out at room temperature. The highest yields were obtained using CABS at pH 118 (178.48 ng DNA), CAPS at pH 11.8 (177.78 ng DNA), CHES at pH 11.8 (178.88 ng DNA) and 2-amino-1-propanesulfonic acid at ph 11.6 (176.90 ng DNA).

### Example 4: Choice of dry storage media

An experiment was carried out to determine the effectiveness of the elution method described here, to elute DNA from a variety of storage media. The folllowing media were tested: S&S IsoCode® paper (Schleicher and Schuell, Keene NH); Gentra Generation® Capture Cards (Gentra Systems, Minneapolis, MN); Gentra Generation® Capture Columns (Gentra Systems, Minneapolis MN); and FTA® paper (Fitzco, Plymouth MN). In order to be able to compare results between experiments, samples of 10 µl of human blood from the same blood lot used for experiments described above, were applied to the IsoCode® paper, Gentra Generation® capture cards, and FTA® paper according to manufacturer's instructions. Each sample was washed using the standard protocol described above. For samples containing the same amount of blood, the Gentra Generation@ Capture Cards provided an average yield of 126± 12 ng of DNA (10 samples) and the FTA® paper provided an average yield of 112 ± 13 ng of DNA (5 samples). S&S IsoCode® paper provided a yield of around 25 ± 10 ng DNA (5 samples), but it should be noted that this DNA yield from S&S IsoCode® paper was difficult to quantitate, due to a high background reading that was presumably due to heme that co-eluted with the DNA.

In an additional experiment, Gentra Generation@ Capture Cards containing the same amount of blood (10 µl) from the same blood lot, were washed using either the manufacturer's instructions (Gentra protocol) or the GenVault protocol provided herein (see Examples 1 or 2), and DNA yields were compared. As noted above, Gentra Generation® Capture Cards washed using the Gentra protocol gave an average yield of 126 ± 12 ng (10 samples). In the present experiment, Gentra Generation® Capture Cards washed with the GenVault protocol gave an average yield of 113 ± 38 ng (10 samples) from samples with the same amount of blood. It should be noted that, when the Gentra Generation® Capture Cards were washed using the GenVault protocol, the cards fell apart into their constituent layers.

The Gentra Generation® Capture Column was used in a format in which 200 µl of blood are loaded on a column, as opposed to the "card" or paper format (*e.g*., FTA®) which was suitable for eluting DNA from 10 µl of blood. The Gentra Generation® Capture Column yielded 929 ± 70 ng from 200 µl of blood. The Qiagen Kit (QiaAmp DNA Blood Mini Kit 50 cat# 51104), using 200 µl of human blood, yielded 3.6 µg of DNA (1 sample). Conditions were changed in an attempt to optimize yield from the Gentra Capture® Column, and an optimized protocol yielded 3.1 +/- .1606 µg from 200 µl of blood (4 samples), or about 85% of the yield obtained using the Qiagen kit.

### Example 5. Elution of DNA from paraffin-embedded tissues

Human breast tissue which had previously been formalin-fixed and paraffin-embedded using standard methods for preparation of histological specimens, was thin-sectioned to produce slices 10 µm thick. Three slices were placed in a tube. To elute DNA, 0.2 ml of GenVault elution buffer (10 mM Tris, pH 11) plus 1% SDS were added and mixed with the tissue sample, followed by heating to 80°C for 10 minutes, after which the mixture was allowed to cool. A ring of hardened paraffin formed on top of the cooled mixture. A syringe was used to pierce the paraffin ring and remove solution (eluate) from the tube. The eluate was neutralized by addition of HCl to a pH of about 8, and either subjected to standard solution phase DNA purification according to the Qiagen protocol (Qiagen Inc., Valencia CA) or alternatively, was applied directly to a 6 mm disk of FTA® paper for storage and air-dried for storage. Purified DNA was obtained from the dried FTA®-bound sample by first using the FTA® washing procedure recommended by the manufacturer, followed by room temperature elution of DNA from the FTA® using 10 mM Tris, pH 11.5, as described above. DNA yield from samples purified by both methods (the Qiagen method and room temperature elution from FTA®) was determined by quantitative PCR to produce a 205 bp amplicon product, using external quantitative human DNA standards (Promega, Inc.) to determine relative yield. By reference to the external standards, it was determined that about 20 µg of DNA was obtained from the sum of three sections, using either the Qiagen purification method or room temperature elution from FTA®.

Additional PCR experiments were carried out using DNA that was purified from paraffin-embedded formalin-fixed tissue sections as described above. These DNA samples supported PCR reactions that produced amplicons as long as about 558 bp.

### Example 6. Elution of GenCode DNA stored with sample

Two (2) GenCodes that differ at only two positions (Code #1 and Code #2), were added to a sample of genomic DNA on dried storage media. The GenVault protocol for room temperature elution was used as described above to elute nucleic acids from the storage media, such that both GenCode and the genomic DNA were eluted. One-thirtieth (1/30^{th}) of the eluted sample was use in a PCR reaction carried out under the following conditions: 16mM (NH₄)₂SO₄, 67 mM Tris-HCl (pH 8.8 at 25°C), 0.01% Tween 20,1.5mM MgCl₂, 200µM of each dNTP (Bioline, Randolph, MA), 5 nM of each GenCode primer, 2.5 units of Biolase (Bioline, Randolph, MA). PCR cycling conditions were as follows: 93°C for 2 minutes, 55°C for 1 minute, 72°C for 2 minutes, followed by 29 cycles of 93°C for 30 seconds, 55°C for 30 seconds, 72°C for 45 seconds.

PCR products were separated on a 6% acrylamide gel (Invitrogen precast 6% TBE gel 1.0mm x 15 well, Cat #EC62655) run in 1X TBE (Apex 10X liquid Tris-Borate-EDTA cat # 20-196) at 130 mV for 45 minutes. Products were visualized by staining the gel with 200 µl TBE containing 20 µl 10,000X Syber Gold (final dilution 1: 100,000) (Molecular Probes, Eugene OR), for 30 minutes shielded from light.

As shown in Figure 4, the GenVault room temperature elution protocol eluted the nucleic acids from each sample on storage media, and PCR of the eluted nucleic acids in each sample using GenCode primers generated clear identifying signatures that corresponded to the GenCode identifier present in each sample: the differences between Code #1 and Code #2 are visible in lanes 4 and 6.

### Example 7. Use of magnetic beads to Purify room temperature eluted DNA

The GenVault room temperature elution protocol provided herein was used in combination with magnetic bead technology for selective extraction of DNA from storage media. In the following example, DNA from 10 µl human blood samples (commercially available "BRT" blood) spotted on FTA® elements was eluted and recovered using two different commercially available magnetic bead systems. Use of magnetic beads provided a simpler protocol for DNA elution and recovery, leading to higher DNA concentrations in the eluate solution(s), higher total DNA recovery, and higher purity of recovered DNA.

### DNA purification using CST™magnetic beads

Selective extraction of DNA from blood spotted on FTA® elements elution was carried out using the GenVault room temperature elution method and "Charge Switch™ Technology" (CST™) magnetic beads from DNA Research Innovation Ltd. (DRI, Sittingbourne, Kent, UK). In order to compare the performance of two DRI products, some blood samples were eluted and recovered using reagents from CST™ Forensic DNA Purification Kit (the "CST™ Forensic Kit"), and some blood samples were eluted and recovered using reagents from the CST™ Genomic DNA Purification Kit for Buccal Cells (the "CST™ Buccal Swab Kit").

*Incubation of FTA® elements with proteinase K.* For samples to be analyzed using the CST™ Forensic Kit, 1 µl DRI proteinase K solution was mixed with 100 µl of DRI lysis buffer, and at least one FTA® element was incubated in the proteinase K/lysis buffer solution. For samples to be analyzed using the CST™ Buccal Swab Kit, 1 µl DRI proteinase K solution was mixed with 100 µl of DRI digestion buffer, and at least one FTA® element was incubated in the proteinase K/digestion buffer solution. FTA® elements were incubated with in the proteinase K-containing incubation solution at room temperature for about 30 minutes. Incubation at room temperature for time periods up to an hour was also successful, and did not improve or reduce DNA yield. FTA® elements were completely submerged in the solution during the incubation. After the incubation step, 12 µl of incubation solution was removed for DNA quantitation using PicoGreen® (Molecular Probes, Inc., Eugene OR).

*Elution of DNA from FTA® elements.* DNA was eluted from the FTA® elements using GenSolve elution buffer at a pH of 11.7 ("GenSolve" is Genvault elution buffer, containing 10 mM Tris at a pH of between 11.5 and 11.7, where the pH is adjusted with NaOH). Using standard protocols, *e.g.*, as described in Examples 1-5 above, the DNA on the FTA® element was eluted 2 to 3 times by adding about 150 to 200 µl GenSolve elution buffer each time. All solutions, including proteinase K incubation solution (about 100 µl) and all eluates (300 to 600 µl) were pooled, and 12 µl was removed for DNA quantitation using PicoGreen® as described above.

*DNA purification using CST™ magnetic beads from DRI.* About 100 µl of a 10% detergent solution was added to the pooled solution (incubation and eluates). The 10% detergent solution supplied by DRI was used successfully. A 10% solution of Triton X-100 also worked, although the final amount of DNA recovered using Triton was about 10-15% less than when the DRI detergent solution was used. After adding the detergent solution, 100 µl of DRI purification buffer was added, and the solution was mixed by pipetting.

Twenty (20) µl of CST™ magnetic beads (DRI ChargeSwitch™ Technology) was added to the solution and mixed by pipetting. The solution was incubated with CST™ beads for 1 to 2 minutes at room temperature, after which the tube containing the solution and beads was placed on/in a magnetic stand to pellet the beads at the bottom of the tube. After about a minute, the solution was carefully aspirated without disturbing the beads. The beads were washed by adding 500 µl DRI wash solution and mixing. For thorough washing, care was taken to separate beads that had formed clumps. After mixing, the tube with beads in wash solution was placed in/on a magnetic stand to pellet the beads. After about a minute, the wash solution was carefully aspirated without disturbing the beads. The wash step was repeated, for a total of two washes of the magnetic beads.

*Elution of DNA from CST™ beads.* After all wash buffer was removed from the pelleted beads, 50 µl of DRI elution buffer was added, and the beads were incubated with CST™ elution buffer for about 2 minutes at room temperature. The tube with beads in CST™ elution buffer was placed in/on a magnetic stand to pellet the beads. After about 1 minute, the CST™ elution buffer was carefully aspirated and 12 µl was removed for DNA quantitation using PicoGreen®.

### DNA purification using Promega MagneSil™ magnetic beads

Selective extraction of DNA from blood spotted on FTA® elements elution was carried out using the GenVault room temperature elution method and MagneSil™ magnetic beads available from Promega Corporation, as described below.

*Incubation of FTA® elements with proteinase K.* Each FTA® element containing a BRT blood sample incubated with 1 µl proteinase K (from DRI CST™ kits described above) and 100 µl of either lysis buffer from the DRI CST™ buccal swab kit, or digestion buffer from the DRI CST™ forensic kit (see descriptions above). FTA® elements were incubated with proteinase K in the incubation buffer for 1 hour at room temperature, or for 15 minutes at 37°C. A 12 µl aliquot was removed for DNA using PicoGreen®.

*Elution of DNA from FTA® elements.* Each FTA® element was removed from the incubation solution, placed in a fresh tube, and eluted 2 to 3 times. For each elution step, 200 µl of GenVault GenSolve elution buffer (10 mM Tris) at a pH of about 11.7 was added to the tube containing the FTA element, mixed for 10 minutes by agitation and then centrifuged at 3500 rpm for 2 minutes. In some experiments, the eluate was aspirated after centrifugation, and the FTA® element was eluted 1 or 2 more times using 200 µl fresh elution buffer each time. In other experiments, the FTA® element was re-eluted using the same elution buffer, where the tube was agitated for 10 minutes and centrifuged in the same solution for 1, 2, or 3 elution cycles. Eluants were aspirated and pooled if necessary. A 12 µl aliquot was removed for DNA using PicoGreen®.

*DNA purification using MagneSil™ magnetic beads.* About 20 µl 10% Triton X-100 detergent solution, 200 µl eluant, 800 µl Promega Lysis Buffer KF, and 200 µl MagneSil™ Paramagnetic Particles (MagneSil™ magnetic beads) were combined in a 2ml sealable tube (e.g., an Eppendorf tube). The stock bottle containing MagneSil™ magnetic beads was mixed (vortexed) before removing a sample. The tube was inverted to mix the beads and solutions, and was incubated for 5 minutes at room temperature with occasional mixing (inversion).

The tube containing solution and beads was placed in/on a magnetic stand to pellet the beads at the bottom of the tube. After about a minute, the solution was carefully aspirated without disturbing the beads. The beads were washed by adding 1000 µl Sat Wash Solution supplied by the manufacturer, followed by mixing. For thorough washing, care was taken to separate beads that had formed clumps. After mixing, the tube with beads was placed in/on a magnetic stand to pellet the beads, and the wash solution was carefully aspirated without disturbing the beads. The beads were then washed twice by adding 1000 µl alcohol wash, mixing, aspirating the wash solution, and repeating.

*Elution of DNA from MagneSi*/*™ beads.* After all alcohol wash was removed from the pelleted beads, 200 µl of nuclease-free water was added to elute DNA from the beads. The tube with beads was inverted for mixing, and then incubated in a water bath for about 5 to 10 minutes at 60-65°C. The tube was removed from the water bath, vortexed briefly, and immediately placed in/on a magnetic stand to pellet the beads. The eluate was removed and a 12 µl aliquot was removed to quantitate DNA with PicoGreen®.

**Table 2. DNA Yields Using CST™ and MagneSil™ Magnetic Beads to Purify Eluted DNA**

| **Method of Recovering Eluted DNA** | **Yield, ng DNA/ml of eluate** | **Yield, total ng DNA recovered** |
|---|---|---|
| **CST™ Forensic Kit** | 3420.1 | 171.01 |
| | 3518.1 | 175.91 |
| | 6837.3 | 341.87 |
| | 4598.9 | 229.95 |
| | 3493.0 | 174.7 |
| | 5539.3 | 276.97 |
| | 3009.0 | 150.50 |
| | 3644 | 182.20 |
| | 3162.7 | 158.14 |
| | 1523.8 | 76.19 |
| | 1495.6 | 74.78 |
| | 3170.2 | 158.51 |
| **Average Yield, CST™ Forensic Kit** | **3617.8** | **180.89** |
| **CST™ Buccal Swab Kit** | 2438.7 | 121.94 |
| | 5369.3 | 268.47 |
| | 4595.5 | 229.78 |
| | 2694.1 | 148.21 |
| | 3392.7 | 169.64 |
| | 2149.1 | 107.46 |
| | 4634.5 | 231.73 |
| | 2589.1 | 129.46 |
| | 3041.3 | 152.07 |
| | 4607 | 230.35 |
| **Average Yield, CST™ Buccal Swab Kit** | **3578.1** | **178.91** |
| **Promega MagneSil™ Beads** | 481.27 | 192.508 |
| | 495.2 | 198.08 |
| | 296.95 | 118.78 |
| | 385.58 | 154.232 |
| | 441.48 | 176.592 |
| | 311.27 | 124.598 |
| | 765.74 | 306.297 |
| | 780.92 | 312.367 |
| **Average yield, MagneSil™ Beads** | **494.8** | **197.9205** |

In the method described above, both CST™ kits (CST™ Forensic Kit and CST™ Buccal Swab Kit) yielded solutions with approximately seven- to eight-fold higher concentrations of DNA (3618.7 and 3578.1 ng/ml, respectively) than the solutions obtained using MagneSil™ beads (494.8 ng/ml). However, the total amount DNA recovered from FTA® elements using all three magnetic bead technologies methods was comparable.

### Example 8. Comparison of DNA yields using conventional and magnetic bead methods.

In another experiment, a comparison of the DNA yield from three (3) FTA® elements using the "conventional procedure" as described in Experiment 1, above, the MagneSil™ beads as described in Example 7, and the CST™ beads as described in Example 7. Use of the "conventional" method, which involved room temperature elution of DNA from FTA® elements but did not include an additional selective extraction (purification step), recovered a total of 80 ng of DNA from 3 elements, in a final solution having 400 ng/ml DNA. The method using room MagneSil™ beads as described in Example 7, which involved room temperature elution from FTA® elements followed by selective extraction (purification) of DNA using magnetic beads, recovered a total of 100 ng DNA from 3 elements, in a final solution having 490 ng/ml DNA. The method using CST™ beads as described in Example 7, which involved room temperature elution from FTA® elements followed by selective extraction (purification) of DNA using magnetic beads, recovered a total of 166 ng DNA from 3 elements, in a final solution having 3300 ng/ml.

### Example 9. Comparison of purification methods for eluted DNA

Three widely used DNA purification technologies were tested for their performance when used with the GenVault room temperature DNA elution method provided herein. To test each technology, 4 GenVault FTA® elements, each spotted with 10 µl human blood (commercial blood samples as described above), were eluted, and DNA was extracted/purified using one technology. The yield of DNA for each of the 4 elements was calculated in terms of total amount of DNA recovered and DNA concentration of the final solution.

### Use of Qiagen QIAprep columns to purify eluted DNA

Four FTA® elements containing 10 µl human blood per element were eluted separately, and the eluted DNA from each element was purified using QIAprep purification columns and reagents (QIAprep Spin Miniprep Kit, Cat No. 27104, Qiagen Inc., Valencia CA). Each FTA® element containing a blood sample was placed in a tube and 200 µl GenSolve elution buffer (10 mM Tris) at a pH of about 11.7 was added. The FTA® element was mixed with elution buffer for about 10 minutes at room temperature, by repeated pipetting the solution in the tube. The tube was centrifuged and the elution buffer was removed (aspirated). This elution protocol was repeated with 200 µl of fresh elution buffer.

The eluate (approximately 400 µl) from each element was mixed with 1 ml of Buffer PB (supplied by the manufacturer). The mixture, corresponding to the eluate from a single element and 1 ml of Buffer PB, was applied separately to a Qiagen QIAprep Spin Column, for a total of 4 columns. The QIAprep columns were centrifuged at 13,000 rpm for 30 to 60 seconds in a tabletop centrifuge. Each column was washed with 750 µl of Buffer PE provided by the manufacturer, and centrifuged at 13,000 rpm for 1 minute. The column was washed a second time with 750 µl of Buffer PE and centrifuged until all wash buffer was removed.

*Elution of DNA from Qiagen column.* To elute DNA from the Qiagen QIAprep columns, 200 µl of Buffer EB (Elution Buffer, provided by manufacturer) was added to each column, and the column was incubated in Buffer EB for 1 minute. Each column was then centrifuged at 13,000 rpm for 1 minute to collect the DNA-containing eluate. Aliquots of the final solution corresponding to each of the 4 FTA® elements was removed for DNA quantitation with PicoGreen®.

**Table 3. DNA Yield Using Qiagen Columns to Purify Eluted DNA**

| **Sample** | **Yield, ng DNA/ml eluate** | **Yield, total ng DNA** |
|---|---|---|
| **1** | 151.3 | 60.4 |
| **2** | 104.5 | 41.9 |
| **3** | 126.7 | 51.0 |
| **4** | 108.9 | 43.6 |
| **Average** | 122.0 | 49.1 |

During the course of the experiment, it was determined that the PCR purification columns from the Qiagen QIAquick PCR Purification Kit (Cat. No. 28104 or 28106, Quiagen Inc.), with a molecular cut-off size of 20 kb, were not suitable to purify DNA eluted using the GenVault room temperature elution method. FTA® elements with 10 µl human blood were eluted at room temperature as described above, and the eluate was mixed with chaotropic buffer N3 (supplied by manufacturer, contianing guanidine hydrochloride and acetic acid). This mixture was loaded on QIAquick columns and centrifuged for 10 minutes in a tabletop centrifuge at maximum speek. The flow-through was discarded. 50 µl of distilled water were added to the top of the column and allowed to stand for 1 minute. The column was centrifuged for 1 minute and the DNA-containing aqueous solution was removed from the bottom of the tube.

### Use of phenol:chloroform to purify eluted DNA

Four FTA® elements containing 10 µl human blood were eluted separately at room temperature, and the eluted DNA was purified using phenol:chloroform extraction of the eluate. Briefly, each FTA® element was placed in a tube and 200 µl of GenSolve elution buffer (10 mM Tris) at a pH of about 11.7 was added to the tube. The FTA® element was mixed with elution buffer (by pipetting) for about 10 minutes. The tube was centrifuged and the elution buffer was removed (aspirated). This elution protocol was repeated with 200 µl of fresh elution buffer.

Both eluates from each FTA® element pooled (approximately 400 µl), and 500 µl of a mixture of phenol:chloroform;isoamyl alcohol (25:25:1) was added to the tube containing the eluates. The tube containing the mixture was vortexed, taking care to ensure that both phases were thoroughly mixed. The tube was then centrifuged at 13,000 rpm for 1 minute in a tabletop centrifuge to separate phases. The upper phase of each mixture was removed from each tube, and the lower phase was extracted a second time with 500 µl of a mixture of phenol:chloroform;isoamyl alcohol (25:25:1), mixed, centrifuged, and the upper phase removed. All steps involving samples containing phenol were handled with proper safety precautions in a fume hood.

The recovered upper phases of each sample (corresponding to eluate from a single FTA® element) were pooled, and 500 µl of choloform:isoamyl alchol (1:1) was added to the tube containing pooled upper phases to remove all traces of phenol. The tube was centrifuged at 13,000 rpm for 1 minute in a tabletop centrifuge. The upper phase was removed, and 500 µl of choloform:isoamyl alchol (1:1) was added to the remaining lower phase. The tube was centrifuged at 13,000 rpm for 1 minute and the upper phase was removed.

**Table 4. DNA Yield Using Phenol:Chloroform to Purify Eluted DNA**

| **Sample** | **Yield, ng DNA/ml eluate** | **Yield, total ng DNA** |
|---|---|---|
| **1** | 386.4 | 135.2 |
| **2** | 381.6 | 133.6 |
| **3** | 405.3 | 141.9 |
| **4** | 338.8 | 118.6 |
| **Average** | 387.1 | 132.3 |

### Use of Gentra Generation® Capture columns to purity eluted DNA

A total of four (4) FTA® elements containing 10 µl human blood were eluted separately, and the eluted DNA was purified using Gentra Generation® Capture columns. Two GenVault FTA® elements (Element #1 and Element #2) were treated with proteinase K and then eluted using the GenVault GenSolve elution reagent (10 mM Tris, ph 11.7) in the GenVault RT Elution protocol as described above. Two Genvault FTA® elements (Element #3 and Element #4) were not treated with proteinase K prior to elution using the GenVault GenSolve elution reagent in the GenVault RT Elution protocol. The DNA in each eluate was then purified using Gentra Generation® Capture columns according to manufacturer's instructions. The recovered DNA was quantitated using PicoGreen®.

**Table 5. DNA Yield Using Gentra Generation® Capture Columns to Purify DNA**

| **Sample** | **Elution Volume, ml** | **Yield, ng DNA/ml** | **Yield, ng DNA/element** | **Average Yield, ng DNA/element** |
|---|---|---|---|---|
| Element #1, proteinase K, after Gentra column | 0.1 | 121.08 | 12.22 | 11.76 |
| Element #2, proteinase K, after Gentra column | 0.1 | 114.18 | 11.42 | |
| Element #3 before application to Gentra Column | 0.4 | 484.63 | 193.85 | 190.35 |
| Element #4 before application to Gentra Column | 0.4 | 467.14 | 186.86 | |
| Element #3 after Gentra column | 0.2 | 49.99 | 10.0 | 10.07 |
| Element #4 after Gentra column | 0.2 | 50.68 | 10.14 | |

Although the yield of DNA was low, these experiments illustrated that the DNA-containing eluates obtained from GenVault FTA elements using the GenSolve reagents that have been developed for the GenVault RT Elution method provided herein, can be successfully purified using a variety of commercially available kits.

## Claims

1. A method for eluting nucleic acid from a nucleic acid-containing sample on storage media, the method comprising:
incubating a storage media comprising a nucleic acid-containing sample with an elution buffer,
wherein the elution buffer has a pH between 11 and 12,
wherein the incubation is carried out at room temperature, and
wherein the nucleic acid-containing sample has been dried on the storage media, wherein said storage media is a polyester material or a cellulosic material selected from the group consisting of S&S 903 paper, IsoCode paper, FTA paper, and Generation Capture Cards.

2. The method of claim 1, wherein the yield of eluted nucleic acid is about 50% or greater.

3. The method of claim 1, wherein the yield of eluted nucleic acid is about 60% to 90%.

4. The method of claim 1, wherein the yield of eluted nucleic acid is about 70% to 80%.

5. The method of any one of claims 1 to 4, wherein the elution buffer comprises uncharged Tris, uncharged ethanolamine, or an alkylsulfonate moiety connected through a secondary amine linkage.

6. The method of any one of claims 1 to 5, wherein the elution buffer comprises a reagent selected from the group consisting of 1) 4-(cyclohexylamino)-1-butanesulfonic acid (CABS), 2) 3-(cyclohexylamino)-1-propanesulfonic acid (CAPS), 3) 3-(cyclohexylamino-2-hydroxy-1-propanesulfonic acid (CAPSO), 4) 2-(cyclohexylamino) ethanesulfonic acid (CHES), 5) N- (2-hydroxyethyl)piperazine-N'-(3-propanesulfonic acid) (EPPS), 6) N-(2-hydroxethyl)piperazine-N'-(2-ethanesullonic acid (HEPES), 7) 2-(N-morpholino) ethanesulfonic acid (MES), 8) 3-(N-morpholino) propanesulfonic acid (MOPS), 9) piperazine-N,N'-bis (2-ethanesulfonic acid) (PIPES), 10) [(2-hydroxy-1,1-bis[hydroxymethyl]ethyl) amino]-1-propanesulfonic acid (TAPS), 11) ethanolamine, 12) 3-amino-1-propanesulfonic acid, and 13) 2-amino-2-hydroxymethyl-1,3-propanediol (Tris).

7. The method of any one of claims 1 to 6, wherein the elution buffer further comprises DNase or RNase.

8. The method of any one of claims 1 to 7, wherein the elution buffer further comprises a detergent.

9. The method of any one of claims 1 to 8, wherein the elution buffer further comprises a preservative or a stabilizer or both.

10. The method of any one of claims 1 to 9, wherein the method further comprises a washing step prior to the elution step, with a wash buffer comprising a protease.

11. The method of any one of claims 1 to 10, further comprising recovering eluted nucleic acid from the media.

12. The method of any one of claims 1 to 11, wherein the method is performed in between about 30 minutes to about 60 minutes.

13. The method of any one of claims 1 to 12, wherein the nucleic acid-containing sample is selected from the group consisting of cell samples, virus samples, pathogenic eukaryotic microorganism samples, unicellular or mutlicellular organism samples, tissue samples, food samples, clinical samples, forensic samples, agricultural samples, environmental samples, and samples derived from an animal subject.

## Patentansprüche

1. Verfahren zur Elution von Nukleinsäure aus einer Nukleinsäure enthaltenden Probe auf einem Speichermedium, wobei das Verfahren umfasst:
Inkubation eines Speichermediums, das eine Nukleinsäure enthaltende Probe umfasst, mit einem Elutionspuffer,
wobei der Elutionspuffer einen pH zwischen 11 und 12 hat,
wobei die Inkubation bei Raumtemperatur erfolgt, und
wobei die Nukleinsäure enthaltende Probe auf dem Speichermedium getrocknet wurde, wobei das Speichermedium ein Polyestermaterial oder ein zellulosisches Material ist, das aus der Gruppe ausgewählt ist, die aus S&S 903 Papier, IsoCode Papier, FTA Papier und Generation Capture Karten ausgewählt ist.

2. Verfahren nach Anspruch 1, wobei die Ausbeute an eluierter Nukleinsäure etwa 50% oder höher ist.

3. Verfahren nach Anspruch 1, wobei die Ausbeute an eluierter Nukleinsäure etwa 60% bis 90% ist.

4. Verfahren nach Anspruch 1, wobei die Ausbeute an eluierter Nukleinsäure etwa 70% bis 80% ist.

5. Verfahren nach einem der Ansprüche 1 bis 4, wobei der Elutionspuffer ungeladenes Tris, ungeladenes Ethanolamin, oder einen Alkylsulfonatrest, gebunden durch eine sekundäre Aminverknüpfung, umfasst.

6. Verfahren nach einem der Ansprüche 1 bis 5, wobei der Elutionspuffer ein Reagenz umfasst, das aus der Gruppe ausgewählt ist, die aus 1) 4-(Cyclohexylamino)-1-butansulfonsäure (CABS), 2) 3-(Cyclohexylamino)-1-propansulfonsäure (CAPS), 3) 3-(Cyclohexylamino-2-hydroxy-1-propansulfonsäure (CAPSO), 4) 2-(Cyclohexylamino)ethansulfonsäure (CHES), 5) N-(2-Hydroxyethyl)piperazin-N'-(3-propansulfonsäure) (EPPS), 6) N-(2-Hydroxyethyl)piperazin-N'-(2-ethansulfonsäure) (HEPES), 7) 2-(N-Morpholino)ethansulfonsäure (MES), 8) 3-(N-Morpholino)propansulfonsäure (MOPS), 9) Piperazin-N,N'-bis(2-ethansulfonsäure) (PIPES), 10) [(2-Hydroxy-1,1-bis[hydroxymethyl]ethyl)amino]-1-propansulfonsäure (TAPS), 11) Ethanolamin, 12) 3-Amino-1-propansulfonsäure, und 13) 2-Amino-2-hydroxymethyl-1,3-propandiol (Tris) besteht.

7. Verfahren nach einem der Ansprüche 1 bis 6, wobei der Elutionspuffer weiterhin DNase oder RNase umfasst.

8. Verfahren nach einem der Ansprüche 1 bis 7, wobei der Elutionspuffer weiterhin ein Detergenz umfasst.

9. Verfahren nach einem der Ansprüche 1 bis 8, wobei der Elutionspuffer weiterhin ein Konservierungsmittel oder einen Stabilisator oder beides umfasst.

10. Verfahren nach einem der Ansprüche 1 bis 9, wobei das Verfahren vor dem Elutionsschritt weiterhin einen Waschschritt mit einem Waschpuffer umfasst, der eine Protease enthält.

11. Verfahren nach einem der Ansprüche 1 bis 10, das weiterhin die Gewinnung eluierter Nukleinsäure aus dem Medium umfasst.

12. Verfahren nach einem der Ansprüche 1 bis 11, wobei das Verfahren zwischen etwa 30 Minuten und etwa 60 Minuten durchgeführt wird.

13. Verfahren nach einem der Ansprüche 1 bis 12, wobei die Nukleinsäure enthaltende Probe ausgewählt ist aus der Gruppe, die aus Zellproben, Virusproben, Proben pathogener eukaryotischer Mikroorganismen, Proben einzelliger oder vielzelliger Organismen, Gewebeproben, Nahrungsproben, klinischen Proben, forensischen Proben, landwirtschaftlichen Proben, Umweltproben, und Proben, die aus einem tierischen Subjekt stammen, besteht.

## Revendications

1. Procédé permettant d'éluer un acide nucléique à partir d'un échantillon contenant un acide nucléique sur un milieu de stockage, le procédé comprenant :
l'incubation d'un milieu de stockage comprenant un échantillon contenant un acide nucléique avec un tampon d'élution,
dans lequel le tampon d'élution a un pH entre 11 et 12,
dans lequel l'incubation est réalisée à température ambiante, et
dans lequel l'échantillon contenant un acide nucléique a été séché sur le milieu de stockage, où ledit milieu de stockage est un matériau polyester ou un matériau cellulosique choisi dans le groupe consistant en du papier S&S 903, du papier IsoCode, du papier FTA et des cartes Generation Capture.

2. Procédé selon la revendication 1, dans lequel le rendement de l'acide nucléique élué est d'environ 50 % ou supérieur.

3. Procédé selon la revendication 1, dans lequel le rendement de l'acide nucléique élué est d'environ 60 % à 90 %.

4. Procédé selon la revendication 1, dans lequel le rendement de l'acide nucléique élué est d'environ 70 % à 80 %.

5. Procédé selon l'une quelconque des revendications 1 à 4, dans lequel le tampon d'élution comprend du Tris non chargé, de l'éthanolamine non chargée ou un radical sulfonate d'alkyle relié par l'intermédiaire d'une liaison amine secondaire.

6. Procédé selon l'une quelconque des revendications 1 à 5, dans lequel le tampon d'élution comprend un réactif choisi dans le groupe consistant en 1) l'acide 4-(cyclohexylamino)-1-butane sulfonique (CABS), 2) l'acide 3-(cyclohexylamino)-1-propane sulfonique (CAPS), 3) l'acide 3-(cyclohexylamino-2-hydroxy-1-propane sulfonique (CAPSO), 4) l'acide 2-(cyclohexylamino)éthane sulfonique (CHES), 5) le N-(2-hydroxyéthyl)pipérazine-N'-(acide 3-propane sulfonique) (EPPS), 6) le N-(2-hydroxyéthyl)pipérazine-N'-(acide 2-éthane sulfonique) (HEPES), 7) l'acide 2-(N-morpholino)éthane sulfonique (MES), 8) l'acide 3-(N-morpholino)propane sulfonique (MOPS), 9) le pipérazine-N,N'-bis(acide 2-éthane sulfonique) (PIPES), 10) l'acide [(2-hydroxy-1,1-bis[hydroxyméthyl]éthyl)amino]-1-propane sulfonique (TAPS), 11) l'éthanolamine, 12) l'acide 3-amino-1-propane sulfonique et 13) le 2-amino-2-hydroxyméthyl-1,3-propanediol (Tris).

7. Procédé selon l'une quelconque des revendications 1 à 6, dans lequel le tampon d'élution comprend en outre une ADNase ou une ARNase.

8. Procédé selon l'une quelconque des revendications 1 à 7, dans lequel le tampon d'élution comprend en outre un détergent.

9. Procédé selon l'une quelconque des revendications 1 à 8, dans lequel le tampon d'élution comprend en outre un conservateur ou un stabilisant ou les deux.

10. Procédé selon l'une quelconque des revendications 1 à 9, dans lequel le procédé comprend en outre une étape de lavage avant l'étape d'élution, avec un tampon de lavage comprenant une protéase.

11. Procédé selon l'une quelconque des revendications 1 à 10, comprenant en outre la récupération de l'acide nucléique élué à partir du milieu.

12. Procédé selon l'une quelconque des revendications 1 à 11, où le procédé est réalisé entre environ 30 minutes et environ 60 minutes.

13. Procédé selon l'une quelconque des revendications 1 à 12, dans lequel l'échantillon contenant l'acide nucléique est choisi dans le groupe consistant en des échantillons cellulaires, des échantillons viraux, des échantillons de micro-organismes eucaryotes pathogènes, des échantillons d'organismes unicellulaires ou multicellulaires, des échantillons tissulaires, des échantillons d'aliments, des échantillons cliniques, des échantillons médico-légaux, des échantillons agricoles, des échantillons environnementaux et des échantillons dérivés d'un sujet animal.
